# EUROPEAN PATENT APPLICATION

(11) **EP 0 997 315 A1**
(43) Date of publication of application: **03.05.2000**
(21) Application number: 99307329.5
(22) Date of filing: 15.09.1999
(51) Int. Cl.: B41M 5/30, C07C 317/22

(54) **Polycyclic phenol compounds and heat-sensitive recording materials employing them**

(30) Priority: 29.10.1998 JP 30868698
(71) Applicant: Nicca Chemical Co., Ltd., Fukui-shi, Fukui 910-8670 (JP)
(72) Inventor: Makino, Masahiro, Sabae-shi, Fukui 916-0073 (JP); Kinoshita, Hirotaka, Fukui-shi, Fukui 910-0024 (JP)
(74) Representative: Hale, Stephen Geoffrey

(57) **Abstract**

Polycyclic phenol compounds represented by the following general formula (1), developers for heat-sensitive recording materials containing these polycyclic phenol compounds, and heat-sensitive recording materials containing these polycyclic phenol compounds as developing substances are provided.

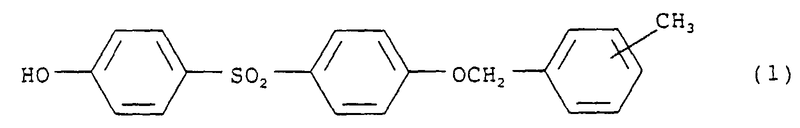

## Description

### BACKGROUND OF THE INVENTION

### 1. Field of the Invention

The present invention relates to novel polycyclic phenol compounds for heat-sensitive recording materials. More specifically, it relates to polycyclic phenol compounds, which are useful as developing substances for heat-sensitive recording sheets, and to heat-sensitive recording materials employing such compounds as developing substances.

### 2. Description of the Related Art

Heat-sensitive recording methods commonly make use of color generation caused by reaction between colorless or light-colored leuco dyes as coloring substances and phenol compounds or organic acid compounds as developing substances and, in such methods, the heat-sensitive recording material is produced by separately dispersing the coloring substance and developing substance prior to adding a binder and mixing, with the addition of a wax, a sensitizer, a surfactant, a defoaming agent, an inorganic pigment or the like if necessary, to prepare a coating solution, and then coating and drying it onto a support such as paper to form a heat-sensitive colored layer.

With recent diversification of heat-sensitive recording systems in this type of heat-sensitive recording method, good performance such as high-speed reactivity, high sensitivity, moisture resistance, plasticizer resistance, good storage stability, etc. has been required depending on the use; however, 4,4'-isopropylidenediphenol (hereunder abbreviated as "BPA"), commonly used in the past, has a melting point of 156-158°C and thus has a high coloration temperature and low sensitivity. Furthermore, despite its high melting point, BPA also has very poor heat resistance, substantial color discharge in printed sections and poor storage stability including moisture resistance and plasticizer resistance, and is prone to discoloration of colored sections. These compounds have also been reported to have an internal secretion disturbing effect, which is undesirable from the standpoint of use as developers. Japanese Examined Patent Publication No. 58-82788 discloses 4-hydroxyphenyl-4'-benzyloxyphenylsulfone (hereunder abbreviated as "SBN") as a developer aimed at increasing coloration density and improving weather resistance, but since the melting point of SBN is within the range of 163-170°C, which is even higher than the melting point of BPA, its coloring sensitivity is even lower rendering it unsuitable for high-speed printing, while a color discharge phenomenon is also seen due to poor oil resistance and plasticizer resistance, and its storage stability is poor.

### BRIEF SUMMARY OF THE INVENTION

It is an object of the present invention to provide heat-sensitive recording materials with low coloration temperature, high coloration density, no deterioration by oils or plasticizers, and satisfactory storage stability.

The present inventors have focused on the fact that the SBN compound described in Japanese Examined Patent Publication No. 58-82788 has disadvantages such as a high melting temperature, inferior high-speed reactivity, proneness to color discharge by oils and plasticizers and poor storage stability, and as a result of diligent research aimed at determining whether these disadvantages could somehow be overcome, have found that compounds according to the present invention do not exhibit such disadvantages and are highly useful; the present invention has been completed on the basis of this finding.

Specifically, the invention provides polycyclic phenol compounds represented by the following general formula (1).

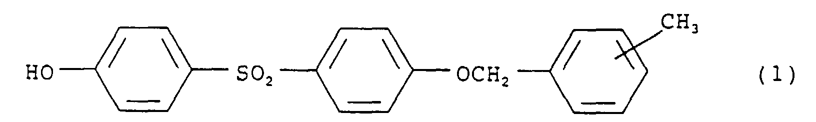

The invention further provides developers for heat-sensitive recording materials, which contain polycyclic phenol compounds represented by general formula (1) above.

The invention still further provides heat-sensitive recording materials comprising supports on which are formed heat-sensitive coloring layers including colorless or light-colored leuco dyes as the coloring substances and developing substances that react with those coloring substances when heated to induce their color generation, the heat-sensitive recording materials being characterized by containing polycyclic phenol compounds represented by general formula (1) above as the developing substances.

### BRIEF DESCRIPTION OF THE DRAWINGS

Fig. 1 is a graph showing the heat coloration properties of recording materials obtained in examples and comparative examples.
Fig. 2 is a graph showing the heat coloration properties of recording materials obtained in other examples and comparative examples.
Fig. 3 is a graph showing the heat coloration properties of recording materials obtained in still other examples and comparative examples.
Fig. 4 is a graph showing the water resistance of printed images obtained in examples and comparative examples.
Fig. 5 is a graph showing the water resistance of printed images obtained in other examples and comparative examples.
Fig. 6 is a graph showing the water resistance of printed images obtained in still other examples and comparative examples.
Fig. 7 is a graph showing the plasticizer resistance of printed images obtained in examples and comparative examples.
Fig. 8 is a graph showing the plasticizer resistance of printed images obtained in other examples and comparative examples.
Fig. 9 is a graph showing the plasticizer resistance of printed images obtained in still other examples and comparative examples.
Fig. 10 is a graph showing the heat resistance of printed images obtained in examples and comparative examples.
Fig. 11 is a graph showing the heat resistance of printed images obtained in other examples and comparative examples.
Fig. 12 is a graph showing the heat resistance of printed images obtained in still other examples and comparative examples.
Fig. 13 is a graph showing the light resistance of printed images obtained in examples and comparative examples.
Fig. 14 is a graph showing the light resistance of printed images obtained in other examples and comparative examples.
Fig. 15 is a graph showing the light resistance of printed images obtained in still other examples and comparative examples.

### DETAILED DESCRIPTION OF THE INVENTION

Polycyclic phenol compounds represented by general formula (1) according to the invention include 4-o-methylbenzyloxy-4'-hydroxydiphenylsulfone, 4-m-methylbenzyloxy-4'-hydroxydiphenylsulfone and 4-p-methylbenzyloxy-4'-hydroxydiphenylsulfone.

4-o-methylbenzyloxy-4'-hydroxydiphenylsulfone (hereunder abbreviated as "SOMB") and 4-m-methylbenzyloxy-4'-hydroxydiphenylsulfone (hereunder abbreviated as "SMMB"), wherein a methyl group is introduced at the ortho position or meta position of a benzyl group, exhibit more excellent water resistance, plasticizer resistance, heat resistance, light resistance and coloration properties than SBN, while 4-p-methylbenzyloxy-4'-hydroxydiphenylsulfone (hereunder abbreviated as "SPMB") has a lower melting point than SBN and therefore exhibits a faster coloration speed and superior water resistance, plasticizer resistance, heat resistance and light resistance. In comparison to BPA, SOMB and SMMB exhibit superior water resistance, plasticizer resistance and heat resistance to BPA, while SPMB exhibits properties roughly equivalent to those of BPA.

As colorless or light-colored Leuco dyes serving as coloring substances which are useful in the heat-sensitive recording materials of the invention there may be mentioned, for example, crystal violet lactone, malachite green lactone, 3,3-bis(p-dimethylaminophenyl)-6-aminophthalide, 3,3-bis(p-dimethylaminophenyl)-6-(p-toluenesulfonamide)phthalide, 3-diethylamino-7-chlorofluoran, 3-diethylamino-7-phenylfluoran, 3-cyclohexylamino-6-chlorofluoran, 3-(N-methyl-N-cyclohexylamino)-6-methyl-7-anilinofluoran and 1,3,3-trimethyl-6'-chloro-8-methoxyindolinobenzospiropyran. These coloring substances may be used alone, or in combinations of two or more.

Conventionally used sensitizers may also be combined with the heat-sensitive recording materials of the invention for the purpose of adjusting the sensitivity, with no restrictions. As such sensitizers there may be mentioned, for example, 2-benzyloxynaphthalene (hereunder abbreviated as "BON"), 1,2-phenoxymethylbenzyl ether, di-p-methylbenzyl oxalate, parabenzyl biphenyl, 1,2-bis-m-methylphenoxyethylene (hereunder abbreviated as "BMPE"), 1,2-bisphenoxyethylene, etc., and these sensitizers may be used alone or in combinations of two or more.

The polycyclic phenol compounds serving as the developing substances for the invention may be used in combination with each other, or they may be used in combination with other developers common in the prior art.

The compounds represented by general formula (1) above according to the invention can be easily synthesized by reacting bisphenol compounds and methylbenzyl chloride by processes which are known to the prior art, and their synthesis can be confirmed by ¹H-nuclear magnetic resonance spectrum (Hereunder abbreviated as ¹H-NMR. An example of a measuring instrument therefor is FT-NMR R-1900 manufactured by Hitachi Laboratories.), infrared absorption spectrum (Hereunder abbreviated as "IR". An example of a measuring instrument therefor is Perkin-Elmer 1650 manufactured by Perkin-Elmer.), melting point (An example of a measuring instrument therefor is a differential scanning calorimeter (hereunder abbreviated as "DSC") manufactured by Shimazu Laboratories.), or the like.

A heat-sensitive recording material employing a compound represented by general formula (1) above according to the invention is produced by a process known to the prior art. For example, it may be obtained by separately dispersing a polycyclic phenol compound of the invention and a coloring substance in a liquid dispersion, dispersing and mixing this into a binder, and coating a support therewith and drying.

The present invention will now be further explained by way of the following examples, with the understanding that the invention is in no way to be limited to these examples. The references to "parts" and "%" throughout the examples indicate parts by weight and percentages by weight.

### Synthesis Example 1

After charging 40.0 g of 4,4'-dihydroxydiphenylsulfone, 200 g of water, 6.7 g of sodium hydroxide and 22.4 g of o-methylbenzyl chloride into a reactor, the mixture was stirred at 60°C for 6 hours. The precipitated reaction product was filtered, and the precipitate was recrystallized with 70% methanol water to obtain 45.1 g of crystals. Both ¹H-NMR and IR analysis of these crystals confirmed them to be the target compound 4-o-methylbenzyloxy-4'-hydroxydiphenylsulfone (SOMB), the melting point of which was 161-164°C (DSC).

### Synthesis Example 2

After charging 40.0 g of 4,4'-dihydroxydiphenylsulfone, 200 g of water, 6.7 g of sodium hydroxide and 22.4 g of m-methylbenzyl chloride into a reactor, the mixture was stirred at 60°C for 6 hours. The precipitated reaction product was filtered, and the precipitate was recrystallized with 70% methanol water to obtain 45.5 g of crystals. Both ¹H-NMR and IR analysis of these crystals confirmed them to be the target compound 4-m-methylbenzyloxy-4'-hydroxydiphenylsulfone (SMMB), the melting point of which was 130-132.6°C (DSC).

### Synthesis Example 3

After charging 40 g of 4,4'-dihydroxydiphenylsulfone, 200 g of water, 6.7 g of sodium hydroxide and 22.4 g of p-methylbenzyl chloride into a reactor, the mixture was stirred at 60°C for 6 hours. The precipitated reaction product was filtered, and the precipitate was recrystallized with 70% methanol water to obtain 44.8 g of crystals. Both ¹H-NMR and IR analysis of these crystals confirmed them to be the target compound 4-p-methylbenzyloxy-4'-hydroxydiphenylsulfone (SPMB), the melting point of which was 133.4°C (DSC).

### Comparative developing substance 1

4-hydroxyphenyl-4'-benzyloxyphenylsulfone (SBN) was used as the comparative developing substance 1.

### Comparative developing substance 2

4,4'-isopropylidenediphenol (BPA) was used as the comparative developing substance 2.

### Examples 1-9, Comparative Examples 1-6

A coloring substance dispersion (solution A), a developing substance dispersion (solution B) and a sensitizer dispersion (solution C), having the compositions listed below, were each separately prepared by dispersion using a sand mill. The combinations of developing substances and sensitizers listed in Table 1 were used to prepare coating solutions for heat-sensitive coloring layers by mixing solution A and solution B in a mixing ratio of 1:2 for Examples 1-3 and Comparative Examples 1 and 2, and solution A, solution B and solution C in a mixing ratio of 1:2:2 for Examples 4-9 and Comparative Examples 3-6; high-quality paper with a weight of 65 g/m² was coated to a dry coating coverage of 8 g/m² and air-dried to fabricate heat-sensitive recording sheets for Examples 1-9 and Comparative Examples 1-6, which were provided for evaluation.

| Solution A (coloring substance dispersion) | |
|---|---|
| PSD-290 (manufactured by Nippon Soda Co., Ltd.) | 1.5 pts. by wt. |
| 10% aqueous polyvinyl alcohol solution | 2.0 pts. by wt. |
| Water | 0.5 pt. by wt. |

| Solution B (developing substance dispersion) | |
|---|---|
| Developing substance | 3.0 pts. by wt. |
| 10% aqueous polyvinyl alcohol solution | 13.0 pts. by wt. |
| Water | 5.5 pts. by wt. |

| Solution C (sensitizer dispersion) | |
|---|---|
| Sensitizer | 3.0 pts. by wt. |
| 10% aqueous polyvinyl alcohol solution | 13.0 pts. by wt. |
| Water | 5.5 pts. by wt. |

**Table 1**

| Solution B Developing substance | Solution C (sensitizer) | | |
|---|---|---|---|
| | None | Bon | BMPE |
| SOMB | Example 1 | Example 4 | Example 7 |
| SMMB | Example 2 | Example 5 | Example 8 |
| SPMB | Example 3 | Example 6 | Example 9 |
| SBN | Comp. Example 1 | Comp. Example 3 | Comp. Example 5 |
| BPA | Comp. Example 2 | Comp. Example 4 | Comp. Example 6 |

### Performance evaluation

### 1. Static coloring property

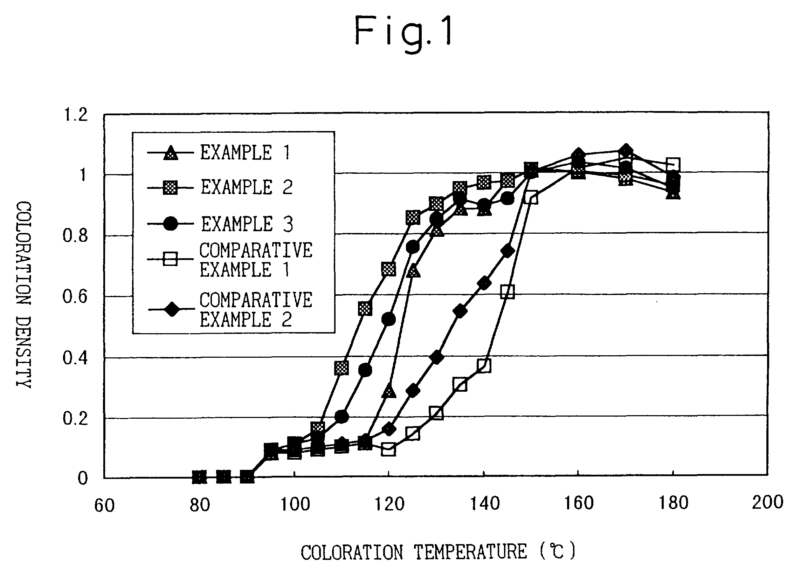

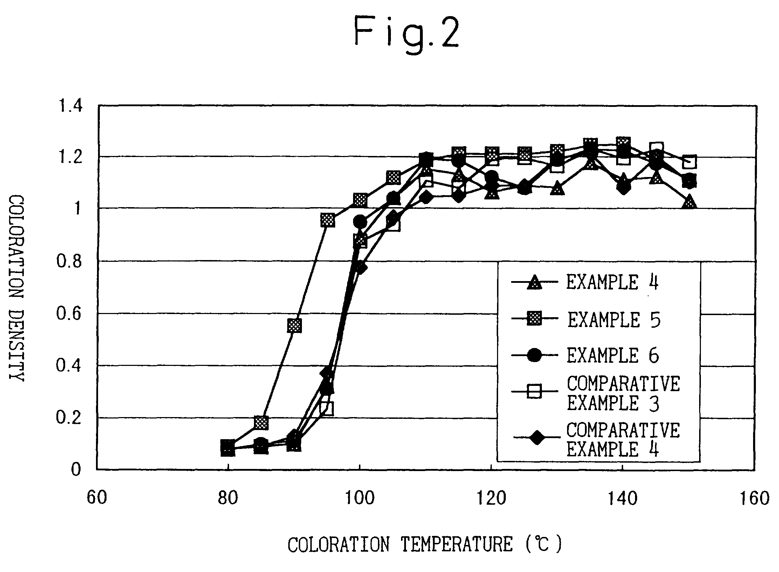

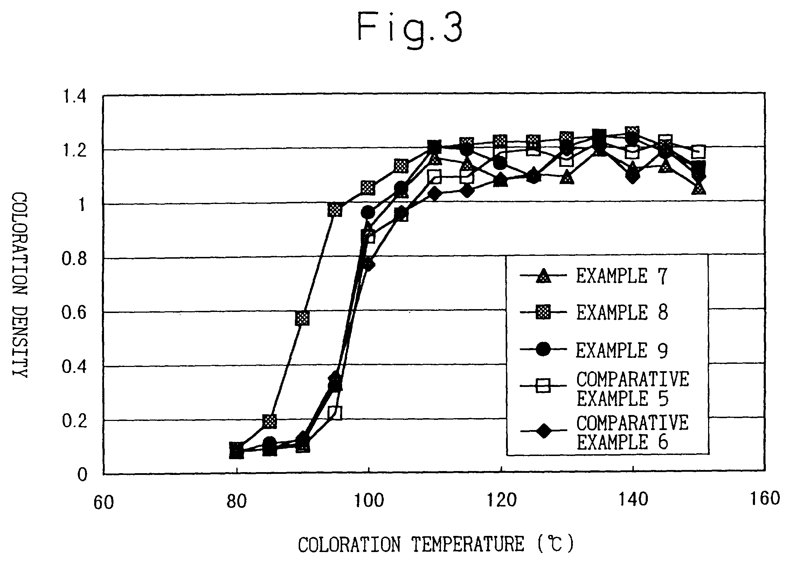

Heated coloration of coated sheets was carried out for 5 seconds at heating temperature gradations of 10°C, under a pressure of 2 kg/cm². The results are shown in Figs. 1-3.

### 2. Water resistance

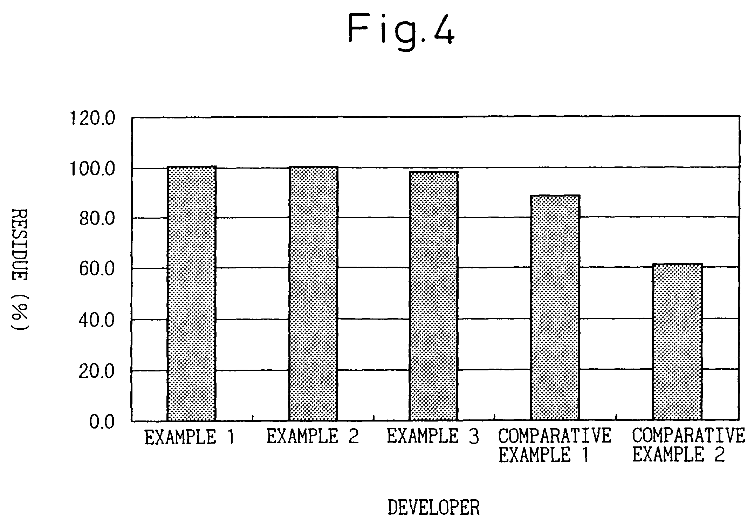

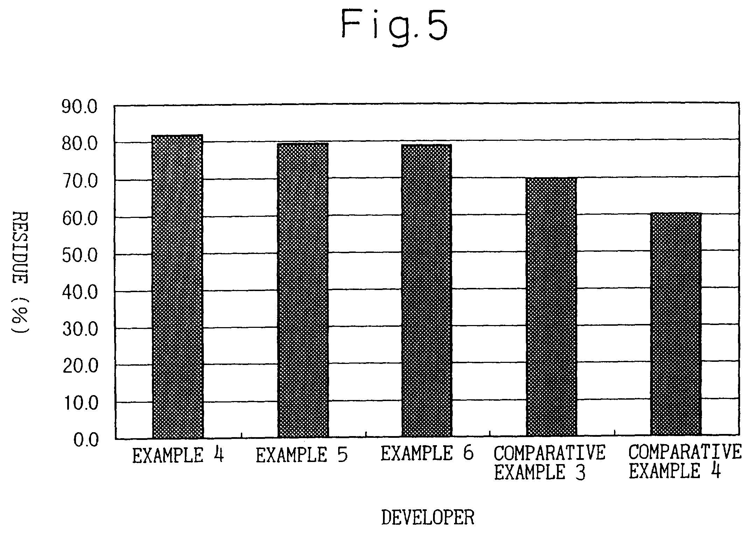

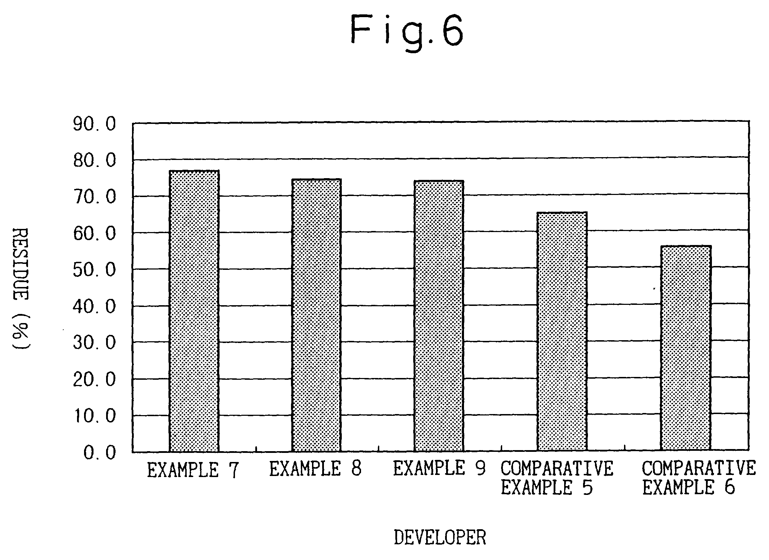

Printed image-formed coated sheets were immersed in water at 20°C for 24 hours, and the residual printing density was measured. The results are shown in Figs. 4-6.

### 3. Plasticizer resistance

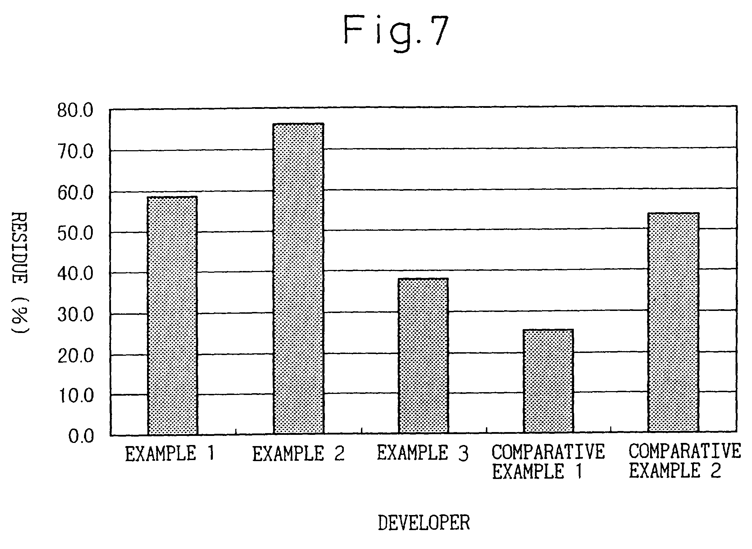

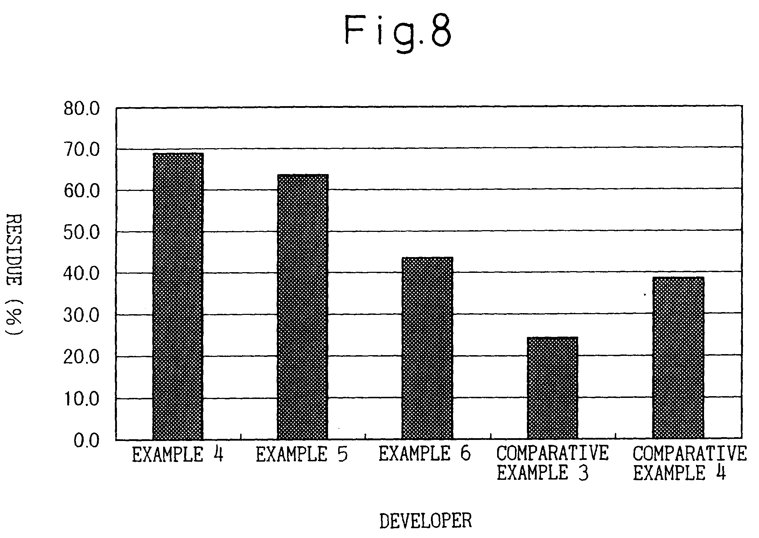

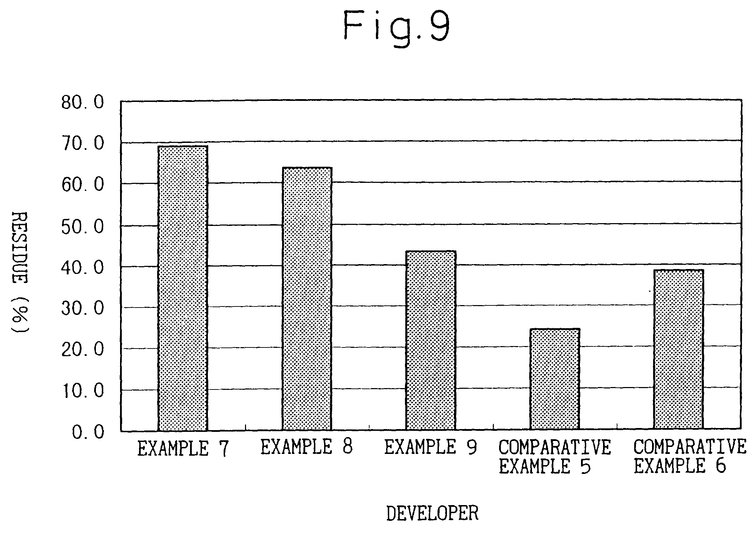

Polyvinyl chloride sheets were pressure-adhered to printed image-formed coated sheets at 50 g/m² and left to stand at 45°C for 24 hours, after which the residual printing density was measured. The results are shown in Figs. 7-9.

### 4. Heat resistance

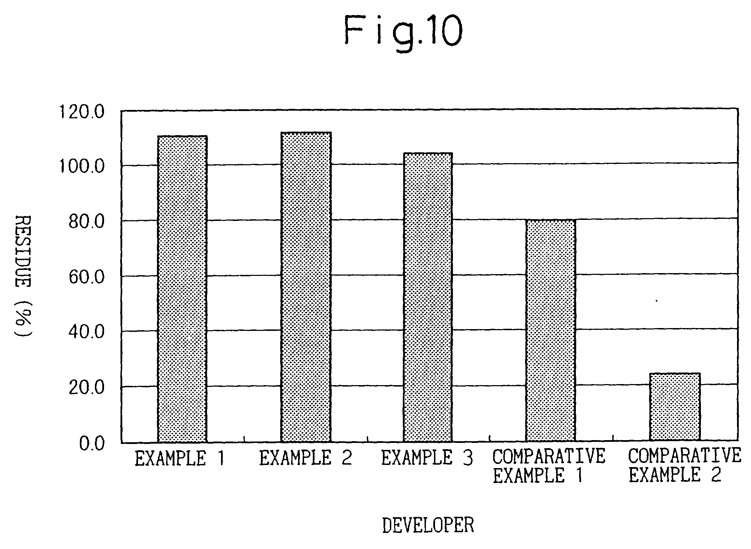

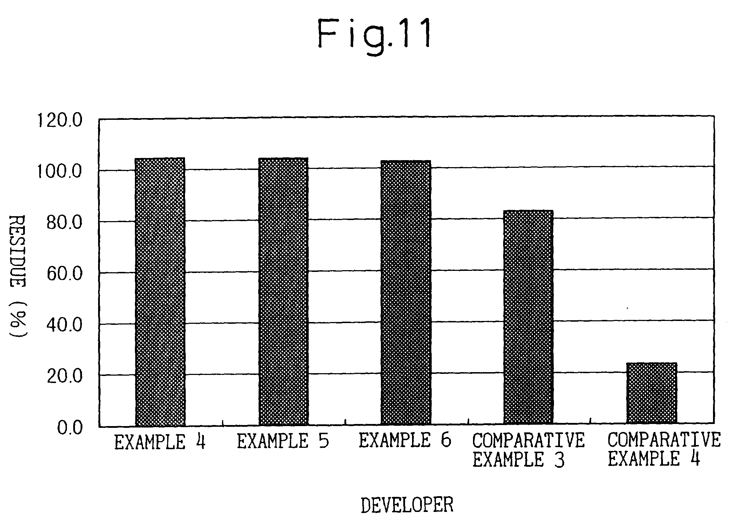

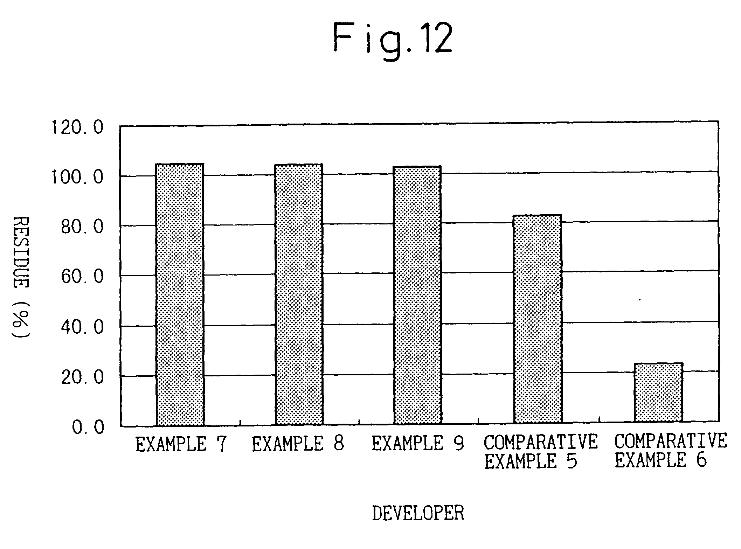

Printed image-formed coated sheets were left to stand dry at 100°C for 24 hours, after which the residual printing density was measured. The results are shown in Figs. 10-12.

### 5. Light resistance

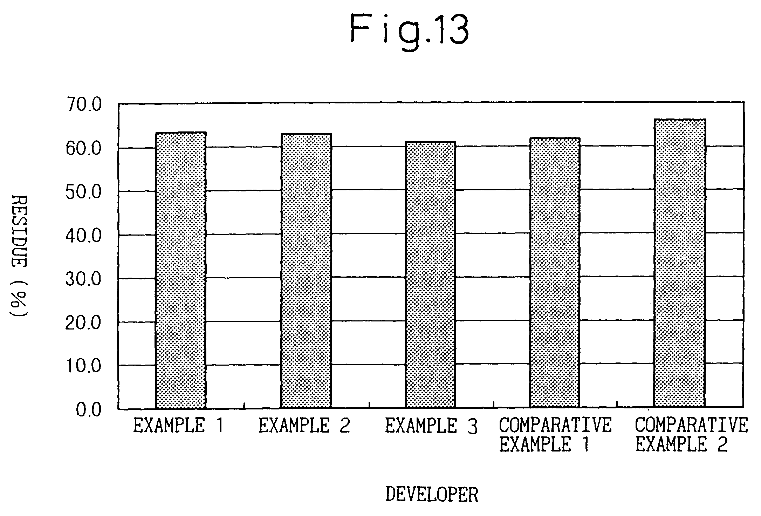

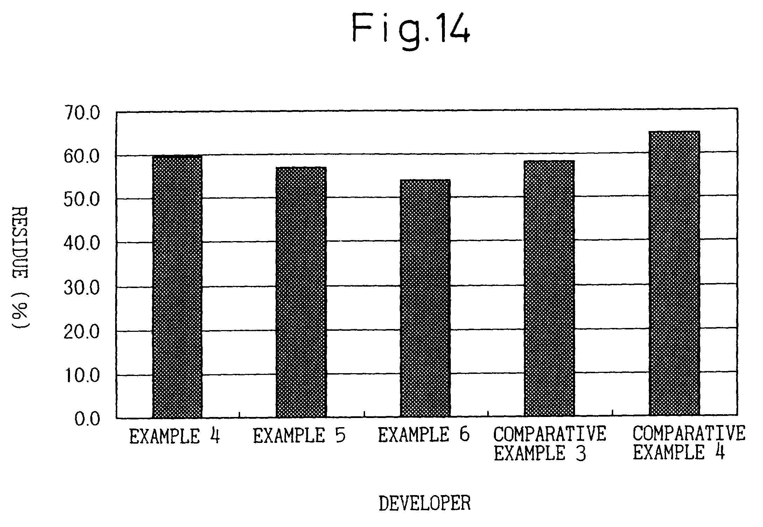

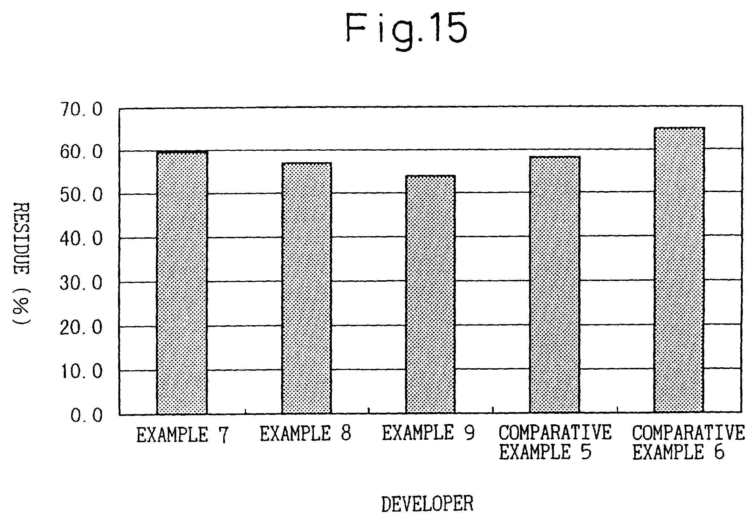

Printed image-formed coated sheets were exposed to light using a fade-o-meter, while dry, at 63°C for 24 hours, after which the residual printing density was measured. The results are shown in Figs. 13-15.

When polycyclic phenol compounds according to the invention are used as developing substances, they generate color at low temperature and thus allow high-speed printing, while also providing heat-sensitive recording materials with satisfactory plasticizer resistance and storage stability.

## Claims

1. A polycyclic phenol compound represented by the following general formula (1):-

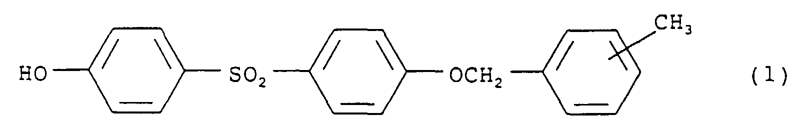

2. A compound according to claim 1 which is selected from the group consisting of 4-o-methylbenzyloxy-4'-hydroxydiphenylsulfone, 4-m-methylbenzyloxy-4'-hydroxydiphenylsulfone and 4-p-methylbenzyloxy-4'-hydroxydiphenylsulfone.

3. A developer for heat-sensitive recording materials, which contains a polycyclic phenol compound according to claim 1.

4. A developer according to claim 3, wherein the polycyclic phenol compound is selected from the group consisting of 4-o-methylbenzyloxy-4'-hydroxydiphenylsulfone, 4-m-methylbenzyloxy-4'-hydroxydiphenylsulfone and 4-p-methylbenzyloxy-4'-hydroxydiphenylsulfone.

5. A recording material which is a heat-sensitive recording material comprising a support on which is formed a heat-sensitive coloring layer including a colorless or light-colored leuco dye as the coloring substance and a developing substance that reacts with said coloring substance when heated to induce its color generation, and which contains a polycyclic phenol compound according to claim 1 as the developing substance.

6. A recording material according to claim 5, wherein the polycyclic phenol compound is selected from the group consisting of 4-o-methylbenzyloxy-4'-hydroxydiphenylsulfone, 4-m-methylbenzyloxy-4'-hydroxydiphenylsulfone and 4-p-methylbenzyloxy-4'-hydroxydiphenylsulfone.

7. A recording material according to claim 5, wherein the colorless or light-colored Leuco dye is selected from the group consisting of crystal violet lactone, malachite green lactone, 3,3-bis(p-dimethylaminophenyl)-6-aminophthalide, 3,3-bis(p-dimethylaminophenyl ) -6- (p-toluenesulfonamide )phthalide, 3-diethylamino-7-chlorofluoran, 3-diethylamino-7-phenylfluoran, 3-cyclohexylamino-6-chlorofluoran, 3-(N-methyl-N-cyclohexylamino) -6-methyl-7-anilinofluoran and 1,3,3-trimethyl-6'-chloro-8-methoxyindolinobenzospiropyran.

8. A recording material according to claim 5, which further contains a sensitizer.

9. A recording material according to claim 8, wherein the sensitizer is selected from the group consisting of 2-benzyloxynaphthalene, 1,2-phenoxymethylbenzyl ether, di-p-methylbenzyl oxalate, parabenzyl biphenyl, 1,2-bis-m-methylphenoxyethylene and 1,2-bisphenoxyethylene.
